# FASCICULE DE BREVET EUROPEEN

(11) **EP 1 802 620 B1**
(45) Date de publication et mention de la délivrance du brevet: **17.12.2008**
(21) Numéro de dépôt: 05804208.6
(22) Date de dépôt: 05.10.2005
(51) Int. Cl.: C07D 471/04, C07C 315/02

(54) **PROCEDE DE PREPARATION ENANTIOSELECTIVE DE DERIVES DE SULFOXYDES.**
VERFAHREN ZUR ENANTIOSELEKTIVEN HERSTELLUNG VON SULFOXIDDERIVATEN
METHOD FOR ENANTIOSELECTIVE PREPARATION OF SULPHOXIDE DERIVATIVES

(30) Priorité: 05.10.2004 FR 0410483
(43) Date de publication de la demande: 04.07.2007
(73) Titulaire: Sidem Pharma S.A., 2441 Luxembourg (LU)
(72) Inventeur: COHEN, Avraham, TEL AVIV (IL); SCHUTZE, François, F-78860 SAINT-NOM-LA-BRETECHE (FR); CHARBIT, Suzy, F-94000 CRETEIL (FR); MARTINET, Frédéric, F-75002 PARIS (FR); GIZECKI, Patricia, F-86000 POITIERS (FR)
(74) Mandataire: L'Helgoualch, Jean
(86) Numéro de dépôt international: PCT/FR2005/002447
(87) Numéro de publication internationale: WO 2006/037894

(56) Documents cités:
- WO-A-03/089408
- WO-A-20/04060891
- US-A- 5 948 789

## Description

La présente invention concerne un procédé de préparation énantiosélective de dérivés substitués de sulfoxydes, et plus particulièrement un procédé de préparation énantiosélective de composés tels que les énantiomères du ténatoprazole et d'autres sulfoxydes comparables.

On connaît divers dérivés de sulfoxydes, et notamment des pyridinyl-méthyl-sulfinyl benzimidazoles, utiles en thérapeutique comme médicaments présentant des propriétés inhibitrices de la pompe à protons, c'est-à-dire des médicaments qui inhibent la sécrétion d'acide gastrique et sont utiles pour le traitement des ulcères gastriques et duodénaux. Le premier dérivé connu de la série des inhibiteurs de la pompe à protons est l'oméprazole, ou 5-méthoxy-2-[[(4-méthoxy-3,5-diméthyl-2-pyridinyl)méthyl]sulfinyl]-1H-benzimidazole décrit dans le brevet EP 005.129, qui possède des propriétés inhibitrices de la sécrétion acide gastrique, et est largement utilisé comme anti-ulcéreux en thérapeutique humaine. D'autres dérivés du benzimidazole à structure similaire sont connus sous leurs noms génériques, par exemple le rabéprazole, le pantoprazole, le lansoprazole, qui présentent tous une analogie structurelle, et se rattachent au groupe des pyridinyl-méthyl-sulfinyl-benzimidazoles.

Le ténatoprazole, c'est-à-dire la 5-méthoxy-2-[[(4-méthoxy-3,5-diméthyl-2-pyridinyl)méthyl]sulfinyl]imidazo[4,5-b]pyridine, est décrit dans le brevet EP 254.588. Il fait aussi partie des médicaments considérés comme des inhibiteurs de la pompe à protons, et il peut également être utilisé dans le traitement du reflux gastro-oesophagien, des hémorragies digestives et des dyspepsies. Cependant, le ténatoprazole se distingue structurellement des autres inhibiteurs de la pompe à protons précités en ce qu'il comprend un noyau imidazo-pyridinyle au lieu d'un noyau benzimidazole.

Tous ces composés sont des sulfoxydes présentant une asymétrie au niveau de l'atome de soufre et peuvent donc se présenter sous forme de mélange racémique de deux énantiomères. Il peut être utile de les séparer sélectivement sous la forme de l'un ou l'autre des deux énantiomères ayant les configurations R et S, ou (+) ou (-), dont les propriétés spécifiques peuvent être sensiblement différentes. Ainsi, la demande de brevet WO 2004060891 décrit l'énantiomère S du ténatoprazole.

Divers procédés ont été décrits dans la littérature scientifique pour préparer de manière sélective ou prépondérante l'un ou l'autre des énantiomères de ces sulfoxydes, en particulier l'oméprazole et son énantiomère de configuration S, l'ésoméprazole, ainsi que ses sels tels que le sel de sodium ou de magnésium.

Ainsi, le brevet EP 652.872 décrit un procédé de préparation du sel de magnésium de l'énantiomère (-) de l'oméprazole par l'intermédiaire de l'ester comportant un groupe acyloxyméthyle chiral, séparation des diastéréo-isomères et solvolyse dans une solution alcaline. Le brevet US 5.776.765 décrit un procédé utilisant la bioréduction stéréosélective du mélange racémique du sulfoxyde en sulfure correspondant, au moyen d'un micro-organisme comprenant une DMSO réductase, permettant d'obtenir un mélange fortement enrichi en énantiomère (-) par rapport à l'énantiomère (+).

H. Kagan et al., ont décrit un système d'oxydation asymétrique de sulfures en sulfoxydes catalysée par un complexe d'isopropylate de titane et de tartrate de diéthyle optiquement actif, en utilisant l'hydroperoxyde de tert-butyle comme oxydant en présence d'eau à une température inférieure à 0°C [voir P. Pitchen et al. J. Am. Chem. Soc. 106, pp. 8188-93 (1984)]. S. Zhao, O. Samuel et H. Kagan, Tetrahedron vol.43, pp. 5135-44 (1987) ont montré que l'énantiosélectivité pouvait être améliorée en utilisant l'hydroperoxyde de cumène dans les mêmes conditions réactionnelles. Diverses variantes de la méthode de Kagan ont été développées et par exemple le brevet US 5.948.789 concerne la préparation énantiosélective de divers sulfoxydes, et plus particulièrement de l'énantiomère (-) de l'oméprazole ou de ses sels de sodium, par oxydation du sulfure correspondant (sulfure "prochiral") par un agent oxydant dans un solvant particulier tel que le toluène et l'acétate d'éthyle, en présence d'une base, la réaction étant catalysée par un complexe de titane obtenu à partir d'un composé de titane^{(IV)}, de préférence l'isopropylate de titane, et d'un ligand chiral choisi parmi des diols aliphatiques et des diols aromatiques, notamment le L(+)- ou le D(-)-tartrate de diéthyle, en présence d'eau. L'addition d'une base au milieu réactionnel permet d'améliorer l'énantiosélectivité de la réaction d'oxydation du sulfure en sulfoxyde. Le procédé décrit dans ce brevet permet d'obtenir un mélange enrichi en l'un ou l'autre des énantiomères (-) et (+), selon le ligand utilisé.

La méthode de Kagan ci-dessus, ainsi que ses variantes, permettent d'obtenir aisément de manière énantiosélective des sulfoxydes ayant une structure du type benzimidazole, tels que l'oméprazole et ses énantiomères. Par contre, dans le cas de sulfoxydes du type imidazo-pyridinyle, la faible solubilité des sulfures dans les solvants classiques tels que le toluène entraîne un milieu réactionnel hétérogène s'accompagnant d'une perte de sélectivité et d'une importante formation de sulfone, de l'ordre de 30%.

Plus particulièrement, certains sulfures, en particulier le sulfure prochiral du ténatoprazole, sont peu solubles dans les solvants usuels tels que le toluène et le chlorure de méthylène, et le choix du solvant pose souvent des difficultés. Ainsi, il est indiqué dans "Asymmetric Catalysis on Industrial Scale", H.U. Blaser, E. Schmidt, 2004 Wiley-VCH Verlag GmbH & Co. KG, Grünstadt, ch. 7, p.413, que les solvants aprotiques et polaires ont un effet défavorable sur l'oxydation asymétrique de sulfures à structure pyridinyl-méthyl-benzimidazole dans des systèmes catalytiques tels que ceux décrits ci-dessus.

Il reste donc souhaitable de pouvoir disposer d'un procédé permettant d'obtenir des énantiomères de sulfoxydes à structure imidazo-pyridinyle avec un excès énantiomérique satisfaisant, en évitant la formation de sulfones, dans de bonnes conditions de rendement et de pureté, en opérant dans un solvant susceptible d'être mis en oeuvre industriellement et présentant une productivité acceptable.

Les travaux effectués par la demanderesse ont permis de montrer que l'on peut préparer des énantiomères de dérivés de sulfoxydes, et en particulier du ténatoprazole et de sulfoxydes à structure similaire, avec un excellent excès énantiomérique, dans de bonnes conditions de rendement, par oxydation énantiosélective du sulfure prochiral correspondant, en présence d'un complexe spécifique à base de titane, sans qu'il soit nécessaire d'ajouter une base au milieu réactionnel.

La présente invention a donc pour objet un procédé de préparation énantiosélective de dérivés de sulfoxydes, et de leurs sels, possédant une asymétrie au niveau de l'atome de soufre, procurant l'un ou l'autre des énantiomères avec une bonne sélectivité et un rendement satisfaisant.

L'invention a tout particulièrement pour objet un procédé de préparation procurant de manière sensiblement énantiosélective l'énantiomère (-) et l'énantiomère (+) du ténatoprazole, et leurs sels. L'expression "de manière sensiblement énantiosélective" utilisée ici signifie que l'on obtient l'énantiomère voulu de manière sélective ou en quantité prédominante par rapport à l'autre énantiomère, c'est-à-dire que l'excès énantiomérique est supérieur ou égal à 90%, de préférence supérieur à 95% et plus particulièrement supérieur à 98%.

Conformément au procédé de l'invention, on effectue une oxydation énantiosélective d'un sulfure de formule générale (I) ci-après

A - CH₂ - S - B (I)

dans laquelle A est un noyau pyridinyle diversement substitué et B un reste hétérocyclique comportant un noyau imidazo-pyridinyle, au moyen d'un agent oxydant en présence d'un catalyseur à base de titane^{(IV)} et d'un ligand chiral constitué par un bêta- ou gamma-amino-alcool cyclique de formule générale (II), dans un solvant organique, suivie le cas échéant d'une salification par une base.

Dans la formule générale (I) ci-dessus, A représente de préférence un groupe pyridinyle ou un groupe pyridinyle portant un ou plusieurs substituants choisis parmi les groupes alkyle linéaire ou ramifié de 1 à 6 atomes de carbone, alcoxy linéaire ou ramifié de 1 à 6 atomes de carbone, méthyle ou éthyle substitué par un ou plusieurs atomes d'halogène, amino, alkylamino ou dialkylamino où la partie alkyle, linéaire ou ramifiée, comporte 1 à 5 atomes de carbone ; B représente un hétérocycle imidazo-[4,5-b]-pyridinyle, substitué le cas échéant par un ou plusieurs groupes alkyle linéaire ou ramifié de 1 à 6 atomes de carbone, alcoxy linéaire ou ramifié de 1 à 6 atomes de carbone, et de préférence substitué sur un ou plusieurs carbones par un groupe méthyle, éthyle, méthoxy ou trihalogénométhyle.

Dans la formule générale (I) ci-dessus, A est de préférence un groupe 2-pyridinyle substitué par un ou plusieurs groupes méthyle, éthyle, méthoxy ou trifluorométhyle, et plus particulièrement un groupe 4-méthoxy-3,5-diméthyl-2-pyridinyle. B est de préférence un groupe 5-méthoxy-imidazo-[4,5-b]-pyridinyle.

Par "bêta- ou gamma-amino-alcool cyclique" dans le cadre de la présente invention, on entend un composé comportant un groupe amino, ou imino, et un groupe hydroxyle ou dérivé, ces deux groupes étant en position bêta ou gamma l'un par rapport à l'autre, et portés par un cycle non aromatique pouvant être lui-même fusionné avec un autre cycle, et contenant éventuellement un ou plusieurs hétéroatomes.

On obtient ainsi un sulfoxyde de formule générale

A - CH₂ - SO - B (Ia)

dans laquelle A et B ont les définitions ci-dessus.

Le sulfure prochiral de départ représenté par la formule (I) ci-dessus est un produit connu qui peut être préparé par diverses méthodes décrites dans la littérature, et par exemple par les méthodes décrites dans les brevets EP 254.588 et EP 103.553.

L'oxydant utilisé dans le procédé de l'invention est de préférence un hydroperoxyde, par exemple l'hydroperoxyde de cumène ou de tertiobutyle. Suivant une forme préférentielle de réalisation, on utilise de l'hydroperoxyde de cumène. Suivant une variante, on peut utiliser comme oxydant une eau oxygénée à concentration élevée, au moins 30%, ou une eau oxygénée complexée par l'urée (UHP ou "urea hydrogen peroxide" H₂NCONH₂.H₂O₂), ci-après également dénommée UHP ; comme indiqué plus loin, la présence d'eau a pour effet de diminuer la sélectivité, mais cet inconvénient peut être au moins en partie compensé par l'addition, dans le milieu réactionnel, d'un agent déshydratant usuel tel que le sulfate de magnésium ou de sodium, ou d'un tamis moléculaire approprié.

Le catalyseur à base de titane est un élément important du procédé de l'invention, qui permet de favoriser la réaction et d'obtenir le dérivé voulu avec un bon rendement. Suivant l'invention, on utilise de préférence un catalyseur tel qu'un complexe de titane^{(IV)}, préparé à partir d'un alcoolate de titane, par exemple l'isopropylate de titane^{(IV)} Ti(OiPr)₄ ou un acétylacétonate de titane. De tels catalyseurs appropriés à base de titane sont décrits dans la littérature et disponibles dans le commerce.

Le choix du ligand constitue un autre élément caractéristique de l'invention car c'est un inducteur de chiralité ; il permet d'orienter sélectivement la réaction vers l'énantiomère voulu. De plus, il conditionne la chimiosélectivité de la réaction.

Suivant le procédé de l'invention, le ligand est un bêta ou gamma amino-alcool cyclique qui peut être représenté par la formule générale (II) suivante : 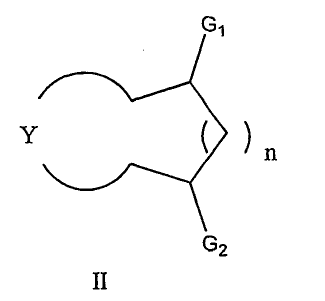 dans laquelle G₁ représente un groupe amino -NR₁R₂ ou un groupe imino -N=CR₁R₂ et G₂ représente un groupe -OR₃, ou inversement ; n est 0 ou 1 ; R₁ et R₂, identiques ou différents, représentent indépendamment l'un de l'autre un atome d'hydrogène, ou un groupe alkyle linéaire ou ramifié de 1 à 6 atomes de carbone ou un groupe aryle, hétéroaryle, acyle ou sulfonyle, R₃ représente un atome d'hydrogène, ou un groupe alkyle linéaire ou ramifié de 1 à 6 atomes de carbone ou un groupe aryle, ou hétéroaryle, et Y est un reste cyclique.

L'amino-alcool cyclique de formule (II) peut être de préférence un dérivé de formule (III) ci-après 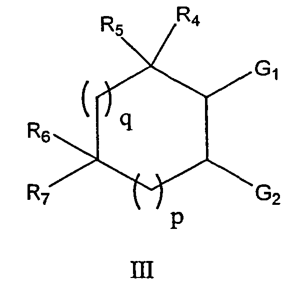 dans laquelle G₁ et G₂ ont les définitions ci-dessus ; p et q, identiques ou différents, sont égaux à 0, 1 ou 2 ; R₄, R₅, R₆ et R₇, identiques ou différents, représentent un atome d'hydrogène ou un groupe alkyle linéaire ou ramifié de 1 à 6 atomes de carbone, R₄ et R₆, et/ou R₅ et R₇, pouvant former ensemble un cycle aliphatique, aromatique ou hétéroaryle, et R₅ et R₇ pouvant former ensemble une liaison simple ou une double liaison avec les carbones les portant.

De préférence, R₄ et R₆, R₅ et R₇, forment ensemble un cycle aromatique, p est 1 et q est 0, pour constituer, par exemple, un dérivé du type amino-indanol.

Dans le cadre de la présente invention :
- un "groupe aryle" signifie de préférence un système mono- ou polycyclique possédant un ou plusieurs noyaux aromatiques parmi lesquels on peut citer le groupe phényle, le groupe naphtyle, le groupe tétrahydronaphtyle, le groupe indanyle et le groupe binaphtyle. Le groupe aryle peut être substitué par 1 à 3 substituants choisis indépendamment les uns des autres parmi un groupe hydroxyle, un groupe alkyle linéaire ou ramifié comportant de 1 à 4 atomes de carbone tel que le méthyle, l'éthyle, le propyle ou de préférence le tert-butyle, un groupe nitro, un groupe (C₁-C₄)alcoxy et un atome d'halogène, tel que le chlore, le brome ou l'iode,
- un "groupe hétéroaryle" signifie de préférence un groupe aryle comportant de 1 à 3 hétéroatomes, tels que l'azote, le soufre ou l'oxygène, et comme tel groupe hétéroaryle on peut citer les groupes pyridinyle, pyrazinyle, pyridazinyle, quinolyle, isoquinolyle, etc,
- un "hétérocycle" ou "groupe hétérocyclique" signifie de préférence un cycle à 5 ou 6 chaînons contenant de 1 à 3 hétéroatomes tels que le soufre, l'azote et l'oxygène. Cette définition contient également les bicycles où un groupe hétérocyclique tel que précédemment défini est fusionné avec un groupe phényle, un groupe cyclohexane ou un autre hétérocycle. Parmi les groupes hétérocycliques on peut citer l'imidazolyle, l'indolyle, l'isoxazolyle, le furyle, le pyrazolyle, le thiényle, etc.

Les ligands de formule générale (II) utilisés dans l'invention présentent une structure moléculaire contrainte, cyclique non aromatique portant les groupes G₁ et G₂, et pouvant être fusionnée avec un cycle. Les essais effectués ont montré que la contrainte de conformation de ce type de ligand est un paramètre majeur favorisant l'énantiosélectivité.

Ainsi, l'amino-alcool optiquement actif utilisé comme ligand dans la présente invention est de préférence un bêta-amino-alcool de stéréochimie relative cis, tel que par exemple le (1*R*,2*S*)-cis-amino-indanol, le 3-exo-diméthylaminobornéol et le cis-2-amino-cyclopentanol.

Plus particulièrement, le ligand de formule (II) peut être notamment le (1*S*,2*R*)-(-)- ou (1*R*,2*S*)-(+)-1-amino-2-indanol. Ainsi, l'utilisation de ce ligand permet d'orienter sélectivement la réaction d'oxydation de la 5-méthoxy-2-[[4-méthoxy-3,5-diméthyl-2-pyridinyl)méthyl]thio]imidazo[4,5-b]-pyridine, pour obtenir sélectivement le *S*-ténatoprazole, comme indiqué ci-après.

Dans les conditions opératoires, le ligand forme avec le catalyseur métallique un complexe asymétrique où le métal est oxydé par l'oxydant.

Suivant une forme de mise en oeuvre du procédé de l'invention, la réaction est effectuée dans un solvant organique, ou dans un mélange de solvants.

Le solvant organique est de préférence aprotique et polaire, et peut être choisi parmi la N-méthyl-pyrrolidone (NMP), le diméthylformamide (DMF), le diméthylacétamide (DMA) et la pyridine, isolément ou en mélange. Les essais effectués ont montré de manière inattendue que ces solvants, dans le cas d'oxydation asymétrique de sulfures comportant un groupe imidazo-pyridinyle, favorisent l'énantiosélectivité et limitent la formation de sulfones.

La réaction d'oxydation s'effectue aisément à froid ou à température ambiante. Il peut être plus avantageux d'effectuer la réaction à une température comprise entre -10 et 30°C et de préférence d'environ 0 à 25°C pour favoriser l'énantiosélectivité. Une température supérieure peut provoquer une baisse de la sélectivité. Par exemple, dans le cas de la préparation du ténatoprazole à partir du sulfure prochiral correspondant, en présence de Ti(OiPr)₄ et d'amino-indanol dans la NMP, l'excès énantiomérique diminue de 97 à 66% quand la température est portée de 20 à 40°C. Au contraire, il est porté à 99% en opérant à froid, à une température voisine de 0°C.

Ainsi, le système catalytique titane / ligand de la présente invention se différentie de ceux des méthodes connues de l'état de la technique tant en ce qui concerne le ligand utilisé que les conditions de mise en oeuvre de la réaction d'oxydation.

L'un des avantages du procédé de l'invention est que l'ordre d'addition des réactifs et composants du milieu réactionnel est indifférent et n'a pas d'incidence notable sur le taux de conversion ni sur l'énantiosélectivité. Suivant une forme préférentielle de mise en oeuvre du procédé, le ligand et le catalyseur à base de titane sont mis en solution dans le solvant pour former le système catalytique titane / ligand, puis le sulfure est ajouté, en solution dans le même solvant, et enfin l'oxydant est ajouté. Suivant une autre variante conforme à l'invention, le système catalytique titane / ligand est ajouté au sulfure en deux étapes, en début et en cours de réaction, c'est-à-dire qu'une nouvelle addition de ligand et de catalyseur au titane est faite en cours de réaction, accompagnée éventuellement d'une nouvelle addition d'oxydant.

Le procédé de l'invention est avantageusement mis en oeuvre dans un solvant en milieu neutre sans nécessiter l'addition d'une base, mais il est préférable d'éviter de travailler en milieu acide qui pourrait entraîner une dégradation du produit final. Les essais effectués ont montré que l'addition d'une base au milieu réactionnel dans les conditions du procédé de l'invention n'améliore pas l'énantiosélectivité et a tendance à diminuer le taux de conversion en sulfoxyde. Ainsi, l'addition de di-isopropyléthylamine a pour effet de diminuer le taux de conversion de plus de 60% à environ 40%. Au contraire, dans un procédé connu comme celui décrit au brevet US 5.948.789 précité, l'addition d'une base telle que la triéthylamine et le N,N-di-isopropyléthylamine améliore l'énantiosélectivité de la réaction.

De plus, la réaction d'oxydation suivant l'invention ne nécessite pas d'eau généralement requise pour améliorer les performances de méthodes classiques. Ainsi, les essais effectués ont montré que, bien au contraire, l'addition d'eau a des effets négatifs sur la sélectivité de la réaction en favorisant la formation de sulfone, sur l'énantiosélectivité en la réduisant fortement, et sur le taux de conversion qui chute de manière importante. Plus particulièrement, les essais réalisés ont montré que la présence d'eau, les autres conditions restant inchangées, a pour effet de diminuer l'excès énantiomérique de 99% à environ 60% tandis que la teneur en sulfone se trouve portée à plus de 7%.

Enfin, la simplification des conditions opératoires de la préparation du complexe titane (IV) / amino-indanol est une part non négligeable de l'intérêt de la présente invention. Suivant une variante du procédé de l'invention, la complexation préalable entre le titane et le ligand chiral peut s'effectuer dans le milieu réactionnel à température ambiante et ne nécessite pas de mûrissement préalable.

Le procédé de l'invention est particulièrement avantageux dans la mesure où l'oxydant et le catalyseur sont largement disponibles dans le commerce, peu coûteux et d'utilisation aisée. De plus, le catalyseur peut être utilisé efficacement en très faible quantité. Le rendement obtenu en énantiomère est excellent, et, de plus, le catalyseur et le ligand peuvent généralement être recyclés dans de bonnes conditions sans perte de l'excès énantiomérique.

Le procédé de la présente invention est tout particulièrement avantageux dans le cas de la préparation des énantiomères du ténatoprazole qui peut être représenté par la formule générale suivante :

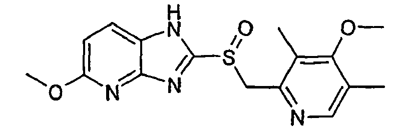

Ainsi, par exemple, suivant le procédé de l'invention, on peut effectuer avantageusement une oxydation énantiosélective de la 5-méthoxy-2-[[(4-méthoxy-3,5-diméthyl-2-pyridinyl)-méthyl]thio]imidazo[4,5-b]pyridine par l'hydroperoxyde de cumène ou l'hydroperoxyde de tert-butyle en présence de Ti(OiPr)₄ et d'amino-indanol dans la NMP, pour obtenir la (-)-5-méthoxy-2-[[(4-méthoxy-3,5-diméthyl-2-pyridinyl)méthyl]-sulfinyl]imidazo[4,5-b]pyridine, c'est-à-dire le ténatoprazole dont la formule est indiquée ci-dessus.

Plus particulièrement, on a constaté que l'oxydation du sulfure ci-dessus permettait d'obtenir l'énantiomère (-), de configuration S, dans d'excellentes conditions de pureté et de rendement si on utilisait un catalyseur à base de titane associé à un ligand constitué par le (1*R*,2*S*)-cis-1-amino-indan-2-ol en solution dans le DMF, la NMP,le DMA ou la pyridine.

Inversement, l'isomère (+), de configuration *R*, peut être obtenu également dans d'excellentes conditions de sélectivité et de rendement en utilisant comme ligand le (1*S*,2*R*)-cis-1-amino-indan-2-ol.

Les énantiomères S et R du ténatoprazole peuvent être utilisés sous forme de sels, notamment de sel de métal alcalin ou alcalino-terreux, et par exemple sous forme de sel de sodium, de potassium, de lithium, de magnésium ou de calcium. Ces sels peuvent être obtenus à partir de l'énantiomère S ou R du ténatoprazole préalablement isolé, par réaction de salification suivant une méthode usuelle de la technique, par exemple par action de réactifs minéraux basiques comportant des contre ions alcalins ou alcalino-terreux.

L'énantiomère S du ténatoprazole correspond à la (-)-5-méthoxy-2-[[(4-méthoxy-3,5-diméthyl-2-pyridinyl)méthyl]sulfinyl]imidazo[4,5-b]pyridine, ou (-)-ténatoprazole. Cette forme peut être déterminée par les mesures de rotation optique suivant les techniques usuelles. Ainsi, l'angle de rotation optique du (-)-ténatoprazole est lévogyre dans le diméthylformamide, et son point de fusion est de 130°C (décomposition).

Les énantiomères S et R du ténatoprazole, dans le traitement des pathologies indiquées ci-dessous, peuvent être administrés sous les formes usuelles adaptées au mode d'administration choisi, par exemple par voie orale ou parentérale, de préférence par voie orale ou intraveineuse.

On peut utiliser par exemple des formulations de comprimés ou de gélules contenant l'un ou l'autre des énantiomères S et R du ténatoprazole comme principe actif, ou encore des solutés buvables ou des émulsions ou solutions pour administration parentérale contenant un sel de ténatoprazole avec un support pharmaceutiquement acceptable usuel. Le sel d'énantiomère du ténatoprazole peut être choisi par exemple parmi les sels de sodium, de potassium, de lithium, de magnésium ou de calcium.

Les énantiomères S et R du ténatoprazole obtenus par le procédé de la présente invention, peuvent être utilisés dans la fabrication de médicaments pour le traitement de pathologies digestives, en particulier celles où une inhibition de la sécrétion acide doit être intense et prolongée, pour le traitement des symptômes et lésions du reflux gastro-oesophagien, des hémorragies digestives résistant aux autres inhibiteurs de la pompe à protons.

La posologie est déterminée par le praticien en fonction de l'état du patient et de la gravité de l'affection. Elle est généralement comprise entre 10 et 120 mg, de préférence entre 20 et 80 mg, d'énantiomère S ou R du ténatoprazole par jour.

Des exemples de préparation d'énantiomères sont décrits ci-après afin d'illustrer la présente invention sans en limiter la portée.

### Exemple 1

### Préparation du (S)-(-)-ténatoprazole

Du (1*R*,2*S*)-(+)-1-amino-2-indanol (22.8 mg, 0.151 mmol) ainsi que de l'isopropylate de titane(IV) (22 µl, 0.076 mmol) sont mis en solution dans la NMP anhydre (0.65 ml).

Une solution dans la NMP de la 5-methoxy-2-[[(4-methoxy-3,5-diméthyl-2-pyridinyl)-méthyl]thio] imidazo[4,5-b]pyridine (100 mg, 0.303 mmol) est ensuite ajoutée au complexe ci-dessus, sous agitation à 20°C, immédiatement suivi par l'addition d'hydroperoxyde de cumène (80%, 65 µl, 0.352 mmol).

Le mélange homogène est maintenu sous agitation à 20°C pendant 5 heures. Le produit brut est constitué de 60% de sulfure, 2% de sulfone et 38% de sulfoxyde avec un excès énantiomérique (e.e.) de 95% (analyse par HPLC chirale).

L'excès énantiomérique est déterminé par chromatographie liquide sous haute pression avec une colonne CHIRALPAK AS 10 µm (250 x 4,6 mm) à 30°C.

| | |
|---|---|
| Eluant : | n-heptane + 0.01% TFA / propan-2-ol (45:55) |
| Débit | 1 ml/min |
| Vol. injecté | 5 µl |
| Longueur d'onde | 302 nm |

Temps de rétention du sulfure 4,3 min
Temps de rétention du R-énantiomère 6,7 min
Temps de rétention de la sulfone 8.1 min
Temps de rétention du S-énantiomère 11,8 min
T_{F} : 129-130°C
[α]²⁰_{D} : -186,6 (c 0,1, DMF)

Spectre UV (méthanol-eau): λₘₐₓ: 272 nm (ε = 6180), 315 nm (ε = 24877).

Infra-rouge (KBr) : 3006, 1581, 1436, 1364, 1262, 1026, 1040 et 823 cm⁻¹.

RMN ¹H (DMSO d₆) δ (ppm) : 2,20 (s, 6H), 3,70 (s, 3H), 3,91 (s, 3H), 4,69-4,85 (m, 2H), 6,80 (d, *J* 8,5 Hz, 1H), 7,99 (d, J 8,5 Hz, 1 H), 8,16 (s, H), 13,92 (s, 1H).

RMN ¹³C (DMSO d₆) δ (ppm) : 13,2 ; 15,0 ; 56,6 ; 60,8 ; 62,6 ; 107,2 ; 129,5 ; 130,4 ; 131,9 ; 135,1 ; 150,5 ; 151,4 ; 156,9 ; 160,7 ; 163,0 ; 166,6.

### Exemple 2

### Préparation du (R)-(+)-ténatoprazole

On procède comme dans l'Exemple 1 en remplaçant le (1*R*,2*S*)-(+)-1-amino-2-indanol par le (1*S*,2*R*)-(-)-1-amino-2-indanol, en faisant agir le même oxydant sur la même quantité de 5-méthoxy-2-[[(4-méthoxy-3,5-diméthyl-2-pyridinyl)méthyl]-thio]imidazo[4,5-b]pyridine que dans l'Exemple 1 et en utilisant le même catalyseur.

Les constantes physiques et spectroscopiques du (R)-ténatoprazole sont identiques à celles du (S)- ténatoprazole, sauf le pouvoir rotatoire spécifique : [α]²⁰_{D} : +185,9 (c 0,1, DMF).

### Exemple 3

### Préparation du (S)-ténatoprazole

le (1*R*,2*S*)-(+)-1-amino-2-indanol (22.8 mg, 0.151 mmol) ainsi que l'isopropylate de titane(IV) (22 µl, 0.076 mmol) sont mis en solution dans le DMF (1 volume). La solution est maintenue à 0°C pendant 30 minutes.

Une solution dans la DMF (6 volumes) de la 5-methoxy-2-[[(4-methoxy-3,5-diméthyl-2-pyridinyl)-méthyl]thio] imidazo-[4,5-b]pyridine (100 mg, 0.303 mmol) est additionnée sous agitation à 20°C, immédiatement suivie par l'addition d'hydroperoxyde de cumène (80%, 65 µl, 0.352 mmol).

Le mélange homogène est maintenu sous agitation à O°C pendant 6 heures. Le produit brut est constitué de 60% de sulfure, 0,1% de sulfone et 39% de sulfoxyde avec un excès énantiomérique (e.e.) de 99% (analyse par HPLC chirale).

### Exemple 4

### Préparation du (S)-ténatoprazole

On procède comme dans l'exemple 3 ci-dessus, mais en remplaçant le DMF par le DMA.

Le mélange homogène est maintenu sous agitation à 22°C pendant 6 heures. Le produit brut est constitué de 47% de sulfure, 0,5% de sulfone et 52% de sulfoxyde avec un excès énantiomérique (e.e.) supérieur à 99% (analyse par HPLC chirale).

### Exemple 5

### Préparation du (S)-ténatoprazole

La 5-methoxy-2-[[(4-methoxy-3,5-diméthyl-2-pyridinyl)-méthyl]thio] imidazo[4,5-b]pyridine (100 mg, 0.303 mmol) est solubilisée dans le DMF anhydre (0.65 ml). L'isopropylate de titane(IV) (22µl, 0.076 mmol) et le (1*R*,2*S*)-(+)-1-amino-2-indanol (22.8 mg, 0.151 mmol) dans le DMF anhydre sont additionnés sous agitation à 22°C, suivi par l'addition d'hydroperoxyde de cumène (80%, 65µl, 0.352 mmol).

Le mélange homogène est maintenu sous agitation à 22°C pendant 5 heures.

Le produit brut est constitué de 61% de sulfure, 1% de sulfone et 38% de sulfoxyde, c'est-à-dire le S-ténatoprazole, avec un excès énantiomérique (e.e.) de 98% (analyse par HPLC chirale).

### Exemple 6

### Préparation du (S)-ténatoprazole

La 5-methoxy-2-[[(4-methoxy-3,5-diméthyl-2-pyridinyl)-méthyl]thio] imidazo[4,5-b]pyridine (100 mg, 0.303 mmol), le (1*R*,2*S*)-(+)-1-amino-2-indanol (22.8 mg, 0.151 mmol) sont solubilisés dans la pyridine anhydre (0.65 ml) ; l'isopropylate de titane(IV) (22 µl, 0.076 mmol) est additionné sous agitation à 22°C, suivi par l'addition d'hydroperoxyde de cumène (80%, 65 µl, 0.352 mmol).

Le mélange homogène est maintenu sous agitation à 22°C pendant 5 heures. Le produit brut est constitué de 16% de sulfure, 4% de sulfone et 68% de sulfoxyde avec un excès énantiomérique (e.e.) de 97% (analyse par HPLC chirale).

L'excès énantiomérique est déterminé par chromatographie liquide sous haute pression avec une colonne CHIRALPAK AS 10 µm (250 x 4,6 mm) à 30°C.

### Exemple 7

### Préparation du (S)-ténatoprazole

La 5-methoxy-2-[[(4-methoxy-3,5-diméthyl-2-pyridinyl)-méthyl]thio] imidazo[4,5-b]pyridine (100 mg, 0.303 mmol) est solubilisée dans la NMP anhydre (0.65 ml), et on y ajoute le (1*R*,2*S*)-(+)-1-amino-2-indanol (22.8 mg, 0.151 mmol) et l'isopropylate de titane(IV) (22 µl, 0.076 mmol) sous agitation à 22°C, suivie par l'addition d'hydroperoxyde de cumène (80%, 65 µl, 0.352 mmol).

Le mélange homogène est maintenu sous agitation à 22°C pendant 5 heures. Le produit brut est constitué de 56% de sulfure, 2% de sulfone et 42% de sulfoxyde avec un excès énantiomérique (e.e.) de 95% (analyse par HPLC chirale).

### Exemple 8

### Préparation du (S)-ténatoprazole

On introduit dans la NMP sous agitation, successivement, la 5-methoxy-2-[[(4-methoxy-3,5-diméthyl-2-pyridinyl)-méthyl]-thio] imidazo[4,5-b]pyridine (0,4 g), puis 0,5 eq. de (1R, 2S)-(+)-cis-1-amino-2-indanol et 0,25 eq. (0,091 ml) de Ti(OiPr)₄ à l'aide d'une seringue en verre de 100µl préalablement séchée en étuve. Puis à la solution, on ajoute, en une seule fois à 0°C, 1,16 eq. (0,26 ml) d'hydroperoxyde de cumène à l'aide d'une seringue (en verre de 250µl préalablement séchée en étuve, en 30 secondes.

La solution homogène est maintenue sous agitation à 0°C pendant 6 heures. A l'aide d'une seringue comme ci-dessus, on ajoute à nouveau, à 0°C, en une seule fois et successivement, 0,25 eq. (0,091 ml) de Ti(OiPr)₄, 0,5 éq. de (1R, 2S)-(+)-cis-1-amino-2-indanol, puis 1,16 eq. (0,26 ml) d'hydroperoxyde de cumène, en 30 secondes et le milieu réactionnel est maintenu sous agitation à 0°C pendant 1,75 heures et maintenu 1 nuit à 5°C au réfrigérateur. En fin de réaction le milieu réactionnel est homogène de couleur jaune orangée.

Le produit brut est constitué de 0,5% de sulfure, 1% de sulfone et 90% de sulfoxyde avec un excès énantiomérique (e.e.) supérieur à 99,5% (analyse par HPLC chirale).

### Exemple 9

### Préparation du (S)-ténatoprazole

On procède comme dans l'Exemple 8 ci-dessus en remplaçant la NMP par le DMF.

Après 5 heures de réaction, on ajoute à nouveau, à 0°C, en une seule fois et successivement, le (1R, 2S)-(+)-cis-1-amino-2-indanol (0,5 éq.), le Ti(OiPr)₄ (0,25 éq.) puis 1,16 eq. (0,26 ml) d'hydroperoxyde de cumène à l'aide d'une seringue en verre de 250µl préalablement séchée en étuve, et le milieu réactionnel est maintenu sous agitation à 0°C.

Des prélèvements sont effectués 5 et 24 heures. En fin de réaction le milieu réactionnel est homogène jaune orangé.

Après 5H00, et avant l'ajout, le produit brut est constitué de 62% de sulfure, 0,1% de sulfone et 38% de sulfoxyde avec un excès énantiomérique (e.e.) supérieure à 99,0% (analyse par HPLC chirale).

Après 24H00, le produit brut est constitué de 13% de sulfure, 2,5% de sulfone et 84% de sulfoxyde avec un excès énantiomérique (e.e.) supérieur à 99,0% (analyse par HPLC chirale).

### Exemple 10

### Préparation du (S)-ténatoprazole

On procède comme dans l'Exemple 9 ci-dessus en remplaçant le DMF par le DMA.

Après 5H00, et avant l'ajout, le produit brut est constitué de 60% de sulfure, 0,8% de sulfone et 38% de sulfoxyde avec un excès énantiomérique (e.e.) supérieur à 99,0% (analyse par HPLC chirale).

Après 24H00, le produit brut est constitué de 3% de sulfure, 4,7% de sulfone et 90% de sulfoxyde avec un excès énantiomérique (e.e.) supérieur à 99,0% (analyse par HPLC chirale).

## Revendications

1. Procédé de préparation énantiosélective de dérivés de sulfoxydes ou de leurs sels, représentés par la formule
A - CH₂ - SO - B (Ia)
dans laquelle A est un noyau pyridinyle diversement substitué et B un reste hétérocyclique comportant un noyau imidazo-pyridinyle,
**caractérisé en ce qu'**il consiste à effectuer une oxydation énantiosélective d'un sulfure de formule générale (I) ci-après
A - CH₂ - S - B (I)
dans laquelle A et B ont les significations ci-dessus,au moyen d'un agent oxydant en présence d'un catalyseur à base de titane^{(IV)} et d'un ligand chiral constitué par un bêta ou gamma-amino-alcool cyclique, représenté par la formule générale (II) suivante : 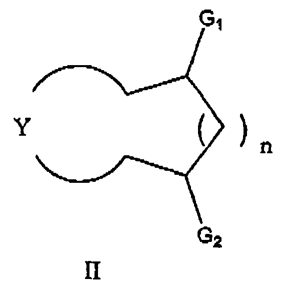 dans laquelle G₁ représente un groupe amino -NR₁R₂ ou un groupe imino -N=CR₁R₂ et G₂ représente un groupe -OR₃, ou inversement ; n est 0 ou 1 ; R₁ et R₂, identiques ou différents, représentent indépendamment l'un de l'autre un atome d'hydrogène, ou un groupe allyle linéaire ou ramifié de 1 à 6 atomes de carbone ou un groupe aryle, hétéroaryle, acyle ou sulfonyle, R₃ représente un atome d'hydrogène, ou un groupe alkyle linéaire ou ramifié de 1 à 6 atomes de carbone ou un groupe aryle, ou hétéroaryle, et Y est un reste cyclique.
dans un solvant organique, suivie le cas échéant d'une salification par une base.

2. Procédé selon la revendication 1, **caractérisé en ce que,** dans la formule générale (I), A représente un groupe pyridinyle ou un groupe pyridinyle portant un ou plusieurs substituants choisis parmi les groupes alkyle linéaire ou ramifié de 1 à 6 atomes de carbone, alcoxy linéaire ou ramifié de 1 à 6 atomes de carbone, méthyle ou éthyle substitué par un ou plusieurs atomes d'halogène, amino, alkylamino ou dialkylamino où la partie alkyle, linéaire ou ramifiée, comporte 1 à 5 atomes de carbone ; B représente un hétérocycle imidazo-[4,5-b]-pyridinyle, substitué le cas échéant par un ou plusieurs groupes alkyle linéaire ou ramifié de 1 à 6 atomes de carbone, alcoxy linéaire ou ramifié de 1 à 6 atomes de carbone.

3. Procédé selon la revendication 2, **caractérisé en ce que** les groupes A et B sont substitués sur un ou plusieurs carbones par un groupe méthyle, éthyle, méthoxy ou trihalogénométhyle.

4. Procédé selon la revendication 3, **caractérisé en ce que** A est un groupe 2-pyridinyle substitué par un ou plusieurs groupes méthyle, éthyle, méthoxy ou trifluorométhyle.

5. Procédé selon l'une quelconque des revendications 3 et 4, **caractérisé en ce que** A est un groupe 4-méthoxy-3,5-diméthyl-2-pyridinyle et B est un groupe 5-méthoxy-imidazo-[4,5-b]-pyridinyle.

6. Procédé selon l'une quelconque des revendications précédentes, **caractérisé en ce que** l'énantiomère obtenu est salifié par action de réactifs minéraux basiques comportant des contre ions alcalins ou alcalino-terreux.

7. Procédé selon la revendication 6, **caractérisé en ce que** le sel est un sel de sodium, de potassium, de lithium, de magnésium ou de calcium.

8. Procédé selon l'une quelconque des revendications 1 à 7, **caractérisé en ce que** l'oxydant est un hydroperoxyde.

9. Procédé selon la revendication 8, **caractérisé en ce que** l'oxydant est l'hydroperoxyde de cumène ou de tertiobutyle.

10. Procédé selon l'une quelconque des revendications 1 à 7, **caractérisé en ce que** l'oxydant est une eau oxygénée à au moins 30%, ou une eau oxygénée complexée par l'urée (UHP), et le milieu réactionnel est additionné d'un agent déshydratant ou d'un tamis moléculaire approprié.

11. Procédé selon l'une quelconque des revendications 1 à 10, **caractérisé en ce que** le catalyseur est un complexe de titane.

12. Procédé selon la revendication 11, **caractérisé en ce que** le complexe de titane est préparé à partir de l'isopropylate de titane ou l'acétylacétonate de titane.

13. Procédé selon la revendication 1, **caractérisé en ce que** l'amino-alcool cyclique est représenté par la formule (III) 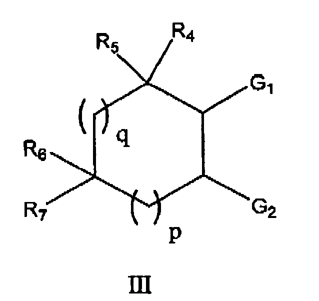 dans laquelle G₁ et G₂ ont les définitions ci-dessus ; p et q, identiques ou différents, sont égaux à 0, 1 ou 2 ; R₄, R₅, R₆ et R₇, identiques ou différents, représentent un atome d'hydrogène ou un groupe alkyle linéaire ou ramifié de 1 à 6 atomes de carbone, R₄ et R₆, et/ou R₅ et R₇, pouvant former ensemble un cycle aliphatique, aromatique ou hétéroaromatique, et R₅ et R₇ pouvant former ensemble une liaison simple ou une double liaison avec les carbones les portant.

14. Procédé selon la revendication 13, **caractérisé en ce que** R₄ et R₆, R₅ et R₇, forment ensemble un cycle aromatique pour constituer un dérivé du type amino-indanol.

15. Procédé selon la revendication 1, **caractérisé en ce que** le ligand est le (1*S*,2*R*)-(-)- ou (1*R*,2*S*)-(+)-1-amino-2-indanol, le 3-exo-diméthylaminobornéol ou le cis-2-amino-cyclopentanol.

16. Procédé selon la revendication 1, **caractérisé en ce que** la réaction d'oxydation est effectuée en solution dans un solvant aprotique polaire choisi parmi la N-méthyl-pyrrolidone (NMP), le diméthylformamide (DMF), le diméthylacétamide (DMA) et la pyridine, isolément ou en mélange.

17. Procédé selon la revendication 1, **caractérisé en ce que** le système catalytique titane / ligand est ajouté au sulfure en deux étapes, en début et en cours de réaction, la deuxième étant accompagnée éventuellement d'une nouvelle addition d'oxydant.

18. Procédé selon l'une quelconque des revendications précédentes, **caractérisé en ce que** l'on effectue une oxydation énantiosélective de la 5-méthoxy-2-[[(4-méthoxy-3,5-diméthyl-2-pyridinyl)méthyl]thio]imidazo[4,5-b]pyridine pour obtenir la (-)-5-méthoxy-2-[[(4-méthoxy-3,5-diméthyl-2-pyridinyl)méthyl]-sulfinyl]imidazo[4,5-b]pyridine en utilisant un catalyseur à base de titane associé à un ligand constitué par le (1*R*, 2*S*)-1-[2-hydroxy-3,5-di-*tert*-butyl-benzylidene)-amino]-indan-2-ol.

19. Procédé selon la revendication 18, **caractérisé en ce que** la réaction d'oxydation est effectuée dans un solvant, en milieu neutre.

20. Procédé selon l'une quelconque des revendications précédentes, **caractérisé en ce que** la réaction est effectuée à une température comprise entre -10 et 30°C.

## Claims

1. Method for the enantioselective preparation of sulfoxide derivatives or of their salts, represented by the following formula:
A - CH₂ - SO - B (Ia)
wherein A is a pyridinyl nucleus diversely substituted and B a heterocyclic residue comprising an imidazo-pyridinyl nucleus,
wherein an enantioselective oxidation of a sulphide of the following general formula (I) is performed
A - CH₂ - S - B (I)
where A and B have the above-mentioned meanings, using an oxidizing agent in the presence of a titanium^{(IV)}-based catalyst and a chiral ligand consisting of a cyclic beta- or gamma-amino-alcohol, represented by the following general formula (II): 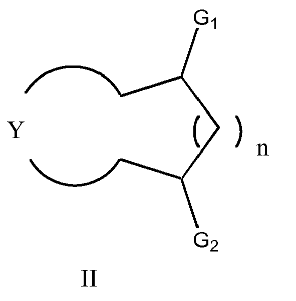 where G₁ represents an amino group -NR₁R₂ or an imino group - N=CR₁R₂ and G₂ represents an -OR₃ group, or the reverse; n equals 0 or 1; R₁ and R₂, identical or different, independently from one another, represent a hydrogen atom or a linear or branched alkyl group of 1 to 6 carbon atoms, or an aryl, heteroaryl, acyl of sulfonyl group, R₃ represents a hydrogen atom, or a linear or branched alkyl group of 1 to 6 carbon atoms or an aryl or heteroaryl group, and Y is a cyclic residue
in an organic solvent, followed as necessary by a salification by a base.

2. Method according to claim 1, wherein in general formula (I), A represents a pyridinyl group or a pyridinyl group bearing one or several substituents chosen among the linear or branched alkyl group of 1 to 6 carbon atoms, linear or branched alkoxy groups of 1 to 6 carbon atoms, methyl or ethyl groups substituted by one or several halogen atoms, amino, alkylamino or dialkylamino groups where the alkyl moiety, linear or branched, comprises 1 to 5 carbon atoms; B represents an imidazo-[4,5-b]-pyridinyl heterocycle, substituted as necessary by one or several linear or branched alkyl groups of 1 to 6 carbon atoms, linear or branched alkoxy groups of 1 to 6 carbon atoms.

3. Method according to claim 2, wherein groups A and B are substituted on one or several carbons by a methyl, ethyl, methoxy or trihalogenomethyl group.

4. Method according to claim 3, wherein A is a 2-pyridinyl group substituted by one or several methyl, ethyl, methoxy of trifluoromethyl groups.

5. Method according to any of claims 3 and 4, wherein A is a 4-methoxy-3,5-dimethyl-2-pyridinyl group and B is a 5-methoxy-imidazo-[4,5-b]-pyridinyl group.

6. Method according to any of the preceding claims, wherein the obtained enantiomer is salified by action of basic mineral reagents comprising alkaline or alkaline earth counter ions.

7. Method according to claim 6, wherein the salt is a sodium, potassium, lithium, magnesium or calcium salt.

8. Method according to any of claims 1 to 7, wherein the oxidizing agent is a hydroperoxide.

9. Method according to claim 8, wherein the oxidizing agent is cumene or tertiobutyl hydroperoxide.

10. Method according to any of claims 1 to 7, wherein the oxidizing agent is hydrogen peroxide at a minimum of 30%, or hydrogen peroxide complexed with urea (UHP), and the reaction medium is supplemented with a dehydrating agent or an appropriate molecular sieve.

11. Method according to any of claims 1 to 10, wherein the catalyst is a titanium complex.

12. Method according to claim 11, wherein the titanium complex is prepared from titanium isopropoxide of titanium acetylacetonate.

13. Method according to claim 1, wherein the cyclic amino-alcohol is represented by the following formula (III) 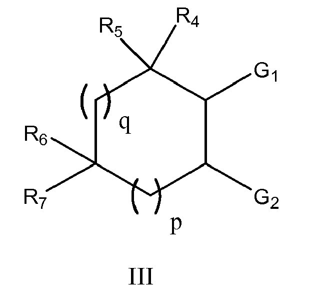 where G₁ and G₂ have the above-mentioned definitions; p and q, identical or different, equals 0, 1 or 2; R₄, R₅, R₆ and R₇, identical or different, represent a hydrogen atom or a linear or branched alkyl group of 1 to 6 carbon atoms, R₄ and R₆, and/or R₅ and R₇, being able to form together an aliphatic, aromatic or heteroaromatic cycle, and R₅ and R₇ being able to form together a simple bond or a double bond with the carbon atoms bearing them.

14. Method according to claim 13, wherein R₄ and R₆, R₅ and R₇, form together an aromatic cycle to constitute a derivative of the amino-indanol type.

15. Method according to claim 1, wherein the ligand is (1*S*,2*R*)-(-)- or (1*R*,2*S*)-(+)-1-amino-2-indanol, 3-exo-dimethylaminoborneol or cis-2-amino-cyclopentanol.

16. Method according to claim 1, wherein the oxidation reaction is performed in solution in a polar aprotic solvent selected from N-methyl-pyrrolidone (NMP), dimethylformamide (DMF), dimethylacetamide (DMA) and pyridine, alone or as a mixture.

17. Method according to claim 1, wherein the titanium / ligand catalytic system is added to sulphur in two steps, at the start of the reaction and during the reaction, the second step being possibly associated to a new addition of oxidant.

18. Method according to any of the preceding claims, wherein an enantioselective oxidation of 5-methoxy-2-[[(4-methoxy-3,5-dimethyl-2-pyridinyl)methyl]thio]imidazo[4,5-b]-pyridine is performed in order to obtain (-)-5-methoxy-2-[[(4-methoxy-3,5-dimethyl-2-pyridinyl)methyl]sulfinyl]imidazo[4,5-b]pyridine using a titanium-based catalyst associated to a ligand constituted by (1*R*, 2*S*)-1-[2-hydroxy-3,5-di-tert-butylbenzylidene)-amino]-indan-2-ol.

19. Method according to claim 18, wherein the oxidation reaction is carried out in a solvent, in neutral medium.

20. Method according to any of the preceding claims, wherein the reaction is carried out at a temperature comprised between -10 and 30°C.

## Patentansprüche

1. Verfahren zur enantioselektiven Herstellung von Sulfoxidderivaten oder Salzen davon, dargestellt durch die Formel
A-CH₂-SO-B (Ia),
worin A ein verschiedenartig substituierter Pyridinylkern ist und B ein heterozyklischer Rest ist, der einen Imidazopyridinylkern umfasst,
**dadurch gekennzeichnet, dass** das Verfahren in der Durchführung einer enantioselektiven Oxidation eines Sulfids der nachstehenden allgemeinen Formel (I)
A-CH₂-S-B (I),
worin A und B wie oben definiert sind, unter Verwendung eines Oxidationsmittels in Gegenwart eines Katalysators auf Basis von Titan^{(IV)} und einem von einem zyklischen β- oder γ-Aminoalkohol gebildeten chiralen Liganden, der durch die nachstehende allgemeine Formel (II) dargestellt ist: 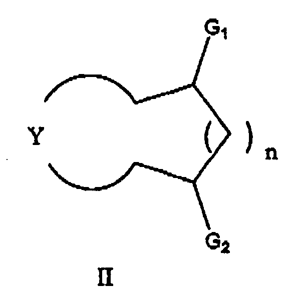 worin G₁ für eine Aminogruppe -NR₁R₂ oder eine Iminogruppe -N=CR₁R₂ steht und G₂ für eine Gruppe -OR₃ steht oder umgekehrt; n = 0 oder 1 ist; R₁ und R₂ gleich oder unterschiedlich sind und unabhängig voneinander für ein Wasserstoffatom oder eine unverzweigte oder verzweigte Alkylgruppe mit 1 bis 6 Kohlenstoffatomen oder eine Aryl-, Heteroaryl-, Acyl- oder Sulfonylgruppe stehen; R₃ für ein Wasserstoffatom oder eine unverzweigte oder verzweigte Alkylgruppe mit 1 bis 6 Kohlenstoffatomen oder eine Aryl- oder Heteroarylgruppe steht; und Y ein zyklischer Rest ist,
in einem organischen Lösungsmittel besteht, worauf gegebenenfalls eine Salzbildung mit einer Base folgt.

2. Verfahren nach Anspruch 1, **dadurch gekennzeichnet, dass** in der allgemeinen Formel (I) A für eine Pyridinylgruppe oder eine Pyridinylgruppe mit einem oder mehreren Substituenten steht, die aus unverzweigten oder verzweigten Alkylgruppen mit 1 bis 6 Kohlenstoffatomen, unverzweigten oder verzweigten Alkoxygruppen mit 1 bis 6 Kohlenstoffatomen, mit einem oder mehreren Halogenatomen substituierten Methyl- oder Ethylgruppen, Amino-, Alkylamino- oder Dialkylaminogruppen, worin der Alkylteil unverzweigt oder verzweigt ist und 1 bis 5 Kohlenstoffatomen umfasst, ausgewählt sind; B für einen Imidazo[4,5-b]pyridinyl-Heterozyklus steht, der gegebenenfalls mit einer oder mehreren unverzweigten oder verzweigten Alkylgruppen mit 1 bis 6 Kohlenstoffatomen oder unverzweigten oder verzweigten Alkoxygruppen mit 1 bis 6 Kohlenstoffatomen substituiert ist.

3. Verfahren nach Anspruch 2, **dadurch gekennzeichnet, dass** die Gruppen A und B an einem oder mehreren Kohlenstoffatomen mit einer Methyl-, Ethyl-, Methoxy- oder Trihalogenmethylgruppe substituiert sind.

4. Verfahren nach Anspruch 3, **dadurch gekennzeichnet, dass** A eine 2-Pyridinylgruppe ist, die mit einer oder mehreren Methyl-, Ethyl-, Methoxy- oder Trifluormethylgruppen substituiert ist.

5. Verfahren nach einem der Ansprüche 3 und 4, **dadurch gekennzeichnet, dass** A eine 4-Methoxy-3,5-dimethyl-2-pyridinylgruppe und B eine 5-Methoxyimidazo[4,5-b]pyridinylgruppe ist.

6. Verfahren nach einem der vorangegangenen Ansprüche, **dadurch gekennzeichnet, dass** aus dem erhaltenen Enantiomer durch das Einwirken von basischen mineralischen Reagenzien, die Alkali- oder Erdalkali-Gegenionen umfassen, ein Salz gebildet wird.

7. Verfahren nach Anspruch 6, **dadurch gekennzeichnet, dass** das Salz ein Natrium-, Kalium-, Lithium-, Magnesium- oder Calciumsalz ist.

8. Verfahren nach einem der Ansprüche 1 bis 7, **dadurch gekennzeichnet, dass** das Oxidationsmittel ein Hydroperoxid ist.

9. Verfahren nach Anspruch 8, **dadurch gekennzeichnet, dass** das Oxidationsmittel Cumol- oder tert-Butylhydroperoxid ist.

10. Verfahren nach einem der Ansprüche 1 bis 7, **dadurch gekennzeichnet, dass** das Oxidationsmittel ein zumindest 30%iges Wasserstoffperoxid oder ein mit Harnstoff komplexiertes Wasserstoffperoxid (UHP) ist und zu dem Reaktionsmedium ein Dehydratisierungsmittel oder ein geeignetes Molekularsieb zugesetzt wird.

11. Verfahren nach einem der Ansprüche 1 bis 10, **dadurch gekennzeichnet, dass** der Katalysator ein Titankomplex ist.

12. Verfahren nach Anspruch 11, **dadurch gekennzeichnet, dass** der Titankomplex ausgehend von einem Titanisopropylat oder einem Titanacetylacetonat hergestellt wird.

13. Verfahren nach Anspruch 1, **dadurch gekennzeichnet, dass** der zyklische Aminoalkohol durch folgende Formel (III) dargestellt ist: 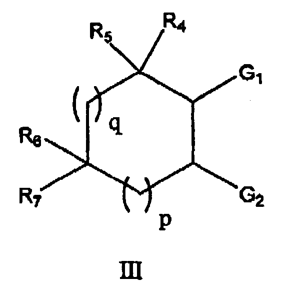 worin G₁ und G₂ wie oben definiert sind; p und q gleich oder unterschiedlich sind und 0, 1 oder 2 sind; R₄, R₅, R₆ und R₇ gleich oder unterschiedlich sind und für ein Wasserstoffatom oder eine verzweigte oder unverzweigte Alkylgruppe mit 1 bis 6 Kohlenstoffatomen stehen, wobei R₄ und R₆ und/oder R₅ und R₇ gemeinsam einen aliphatischen, aromatischen oder heteroaromatischen Ring bilden können und R₅ und R₇ mit den Kohlenstoffatomen, an die sie gebunden sind, eine Einfachbindung oder Doppelbindung bilden können.

14. Verfahren nach Anspruch 13, **dadurch gekennzeichnet, dass** R₄ und R₆, R₅ und R₇ gemeinsam einen aromatischen Ring bilden, um ein Derivat vom Aminoindanol-Typ zu bilden.

15. Verfahren nach Anspruch 1, **dadurch gekennzeichnet, dass** der Ligand (1S,2R)-(-)- oder (1R,2S)-(+)-1-Amino-2-indanol, 3-Exodimethylaminoborneol oder cis-2-Aminocyclopentanol ist.

16. Verfahren nach Anspruch 1, **dadurch gekennzeichnet, dass** die Oxidationsreaktion in Lösung in einem aprotischen polaren Lösungsmittel durchgeführt wird, das aus N-Methylpyrrolidon (NMP), Dimethylformamid (DMF), Dimethylacetamid (DMA) und Pyridin, einzeln oder als Gemisch, ausgewählt ist.

17. Verfahren nach Anspruch 1, **dadurch gekennzeichnet, dass** das Titan/Ligand-Katalysatorsystem in zwei Schritten zu Beginn und im Verlauf der Reaktion zugesetzt wird, wobei die zweite Zugabe gegebenenfalls von einer erneuten Zugabe von Oxidationsmittel begleitet wird.

18. Verfahren nach einem der vorangegangenen Ansprüche, **dadurch gekennzeichnet, dass** eine enantioselektive Oxidation von 5-Methoxy-2-[[(4-methoxy-3,5-dimethyl-2-pyridinyl)methyl]thio]imidazo[4,5-b]pyridin unter Einsatz eines Katalysators auf Basis von Titan in Verbindung mit einem Liganden durchgeführt wird, der aus (1R,2S)-1-[2-Hydroxy-3,5-di-tert-butylbenzyliden)amino]indan-2-ol besteht, um (-)-5-Methoxy-2-[[(4-methoxy-3,5-dimethyl-2-pyridinyl)methyl]-sulfinyl]imidazo[4,5-b]pyridin zu erhalten.

19. Verfahren nach Anspruch 18, **dadurch gekennzeichnet, dass** die Oxidationsreaktion in einem Lösungsmittel in einem neutralen Medium durchgeführt wird.

20. Verfahren nach einem der vorangegangenen Ansprüche, **dadurch gekennzeichnet, dass** die Reaktion bei einer Temperatur zwischen -10 und 30 °C durchgeführt wird.
